# EUROPEAN PATENT SPECIFICATION

(11) **EP 3 727 334 B1**
(45) Date of publication and mention of the grant of the patent: **07.06.2023**
(21) Application number: 18836409.5
(22) Date of filing: 19.12.2018
(51) Int. Cl.: A61K 9/00, A61K 9/06, A61K 31/197, A61K 41/00, A61K 47/10

(54) **PHARMACEUTICAL PREPARATION COMPRISING A TOPICALLY RELEASED ACTIVE INGREDIENT AND A HEAT-SENSITIVE CARRIER, METHOD OF OBTAINING SAME, AND USE OF SAME IN THE TREATMENT OF SKIN OR MUCOSAL INFECTIONS**
PHARMAZEUTISCHES PRÄPARAT MIT EINEM TOPISCH FREIGESETZTEN WIRKSTOFF UND EINEM WÄRMEEMPFINDLICHEN TRÄGER, VERFAHREN ZUR HERSTELLUNG DAVON UND VERWENDUNG DAVON IN DER BEHANDLUNG VON HAUT- ODER SCHLEIMHAUTINFEKTIONEN
PRÉPARATION PHARMACEUTIQUE COMPRENANT UN PRINCIPE ACTIF À LIBÉRATION PAR VOIE TOPIQUE ET UN SUPPORT THERMOSENSIBLE, PROCÉDÉ PERMETTANT DE L'OBTENIR, ET UTILISATION DE CELLE-CI DANS LE TRAITEMENT D'INFECTIONS DE LA PEAU OU DES MUQUEUSES

(30) Priority: 20.12.2017 IT 201700147608
(43) Date of publication of application: 28.10.2020
(73) Proprietor: Alpha Strumenti S.r.l., 20066 Melzo (MI) (IT)
(72) Inventor: ARDEMAGNI, Franco, 20066 Melzo (MI) (IT)
(74) Representative: Simino, Massimo
(86) International application number: PCT/IB2018/060368
(87) International publication number: WO 2019/123332

(56) References cited:
- EP-A1- 2 641 590
- EP-A1- 3 127 557
- WO-A1-2009/105369

## Description

### Field of The Invention

In its basic aspect, the present invention relates to a pharmaceutical preparation comprising a topically released active ingredient and a pharmaceutical carrier with properties including mucoadhesion, thermosensitivity and optimized active ingredient release in the skin and mucosa.

In particular, the present invention relates to a pharmaceutical preparation containing a topically released active ingredient suitable for use in photodynamic therapy, namely 5-aminolevulinic acid and/or its salts or pharmaceutically acceptable derivatives.

The present invention also relates to a pharmaceutical preparation containing an active substance, namely 5-aminolevulinic acid and/or its acid addition salts or pharmaceutically acceptable derivatives, for use in the treatment of pathological conditions or lesions of the skin or the mucosa by photodynamic therapy.

The present invention also relates to a method of obtaining a pharmaceutical preparation as mentioned above as well as the use of a pharmaceutical carrier with properties including mucoadhesion, optimized transdermal release and thermosensitivity in the production of a pharmaceutical preparation as mentioned above for treatment of pathological conditions or lesions of the skin or the mucosa by photodynamic therapy.

### Prior art

Photodynamic therapy (PDT) is known to be based on absorption and/or preferential retention of photosensitive substances by tumor cells. Photosensitive substances are generally inert but are able to stimulate production of toxic substances that can cause cell damage or death and therefore ultimately kill or inactivate tumor cells and pathogenic organisms when exposed to radiation of a given wavelength.

In dermatology, photodynamic therapy consists in the application of a preparation containing δ-aminolevulinic acid or 5-aminolevulinic acid (ALA) in a vehicle allowing topical release thereof in the area of skin to be treated (see for example Thomas J. Dougherty, Charles J. Gomer, Barbara W. Henderson, Giulio Jori, David Kessel, Mladen Korbelik, Johan moan, and Qian Peng (1998) "Photodynamic Therapy" J Natl Cancer Inst. 1998 Jun 17;90(12): 889-905).

The desired biological effects of the therapy derive from interaction between light radiation and Protoporphyrin-9 (PpIX) which is a product of the metabolic conversion of ALA; this molecule is a powerful photo-sensitizer, whereas ALA itself does not interact with light radiation.

Conversion of ALA into PpIX selectively occurs in cells in which the metabolic rate is accelerated by pathological conditions (as in tumors or dysplasias), the presence of inflammation (e.g. due to acne or mechanical stimulation: micro-needling) or in living organisms such as bacteria, in which the metabolic rate is structurally higher; this forms the basis for treatment selectivity.

PpIX converts light radiation into chemical energy by a photochemical process; the biological effects derive from the production of reactive oxygen species (ROS) inside the cell (or bacterium) in which a sufficient concentration of PpIX was determined; if the ROS production rate exceeds a tolerable value it causes apoptosis or necrosis of the target cell, thereby achieving the desired therapeutic action.

In order to obtain an adequate PpIX concentration for the therapeutic action, an incubation time shall be interposed between application of the topical preparation and light irradiation; shorter times will apparently beneficial for therapy execution.

Conventionally, PDT has been strictly used in dermatology for treatment of skin epitheliomas (see for example Stanley B. Brown, Elizabeth A. Brown, Ian Walker (2004) "The present and future role of photodynamic therapy in cancer treatment" Lancet Oncology - Volume 5, No 8 , p497-508, August 2004*;* Morton, C.A., Szeimies, R.-M., Sidoroff, A. and Braathen, L.R., (2013) "European guidelines for topical photodynamic therapy part 1: treatment delivery and current indications - actinic keratoses, Bowen's disease, basal cell carcinoma." JEADV 2013; 27: 536-544; Morton, C.A., Szeimies, R.-M., Sidoroff, A. and Braathen, L.R., (2013) "European guidelines for topical photodynamic therapy part 2: emerging indications - field cancerization, photorejuvenation and inflammatory/infectious dermatoses." JEADV 2013; 27: 672-679) and acne at the active stage (see Stanley B. Brown et al, above).

In recent times, the effectiveness of the therapy was also assessed in different fields, the salient effect being antimicrobial efficiency in the treatment of a wide variety of pathogens (see Harris F1, Pierpoint L. (2012) "Photodynamic therapy based on 5-aminolevulinic acid and its use as an antimicrobial agent." Med Res Rev. 2012 Nov;32(6):1292-327*;* Ludmila M. Baltazar, Anjana Ray, Daniel A. Santo. Patricia S. Cisalpino, Adam J. Friedman and Joshua D. Nosanchuk (2015) "Antimicrobial photodynamic therapy: an effective alternative approach to control fungal infections" Front Microbiol. 2015; 6: 202*;* Felipe F Sperandio,Ying-Ying Huang and Michael R Hamblin (2013) "Antimicrobial Photodynamic Therapy to Kill Gram-negative Bacteria" Recent Pat Antiinfect Drug Discov. 2013 Aug; 8(2): 108-120*;* Mark Wainwright, Tim Maisch, Santi Nonell, Kristjan Plaetzer, Adelaide Almeida, George P Tegos, Michael R Hamblin (2016) "Photoantimicrobials-are we afraid of the light?" Lancet Infect Dis 2016*).* Particularly:
- Wound care applications: photodynamic therapy of skin ulcerations (wound care) as described, for example, in Stan Brown (2012) "Clinical Antimicrobial Photodynamic Therapy: Phase II Studies in Chronic Wounds" JNCCN-Journal of the National Comprehensive Cancer Network Volume 10 Supplement 2 October 2012*;*
- Dental applications: photodynamic therapy of peridontitis and periimplants and other gingival and oral cavity infections as described for instance in S. Rajesh, Elizabeth Koshi, Koshi Philip, and Aparna Mohan (2011) "Antimicrobial photodynamic therapy: An overview" J Indian Soc Periodontol. 2011 Oct-Dec; 15(4): 323-327*;* Tennert C 1 , Feldmann K, Haamann E, Al-Ahmad A, Follo M, Wrbas KT, Hellwig E, Altenburger MJ. ( 2014) "Effect of photodynamic therapy (PDT) on Enterococcus faecalis biofilm in experimental primary and secondary endodontic infections." BMC Oral Health. 2014 Nov 4;14:132*.*

Photodynamic therapy with ALA used as a broad-spectrum antimicrobial agent is advantageous as compared with conventional antibiotics as its action mechanism is active on a wide variety of pathogens and does not induce resistance; this advantage, in addition to the negligible side effects of therapy (ALA is a homologous compound as it is part of the heme synthesis cycle) allows it to be used for effective treatment of infections of the skin (acne, ulcers) and mucosa (gingivitis, periodontitis, etc.).

In these applications, the use of a carrier that allows direct topical release of the active ingredient to the site to be treated and sustained maintenance of the required concentration of the active ingredient are of the utmost importance.

In this respect, mucoadhesive polymer carriers have been used, which can adhere o the epithelial surface or the mucosa and allow direct sustained release of active ingredients (e.g. a drug) to the body site of application.

A class of carriers of this type that has been found to be particularly convenient for topical applications uses triblock copolymers of poly(ethylene oxide)-poly(propylene oxide)-poly(ethylene-oxide) (PEO-PPO-PEO), known as poloxamers which both have mucoadhesion properties and are also thermoreversible (or heat sensitive), i.e. have a solution-to-gel (sol-gel) transition temperature that may be suitably controlled according to the preparation, i.e. because the PEO block has hydrophilic properties and the PPO block has hydrophobic properties.

In addition, poloxamers are commonly used in the production of these thermosensitive gels also due to their toxicological properties (see for example E. A. G. Aniansson; S. N. Wall (1974). "Kinetics of step-wise micelle association". J. Phys. Chem. 78 (10): 1024-1030; P. Alexandridis; T. Alan Hutton (1995) "Polyethylene oxide)-poly(propylene oxide)-poly(ethylene oxide) block copolymer surfactants in aqueous solutions and at interfaces: thermodynamics, structure, dynamics, and modeling". Colloids Surf. A Physicochem. Eng. Asp. 96: 1-46; Bartrakova E.V.; Kabanov A.V. (2008). "Pluronic block copolymers: Evolution of drug delivery concept from inert nanocarriers to biological response modifiers". J. Control. Release. 130 (2): 98-106*.*

Esra Baloglu et al. "Rheological and mechanical properties of poloxamer mixtures as a mucoadhesive base gel", Pharmaceutical Development and Technology, 2011; 16(6): 627-636 describes a study on rheological, mechanical and mucoadhesive properties of thermosensitive formulations containing poloxamer 407 and poloxamer 188 used alone and in combination as carriers for topical release of drugs. In particular, formulations containing poloxamer 407 and poloxamer 188 in a ratio of 15:15 (formulation F5), 15:20 (formulation F6) and 20:10 (formulation F7) have the best sol-gel transition temperature characteristics for administration to the mucosa, the formulation F6 nevertheless exhibiting poor gel properties and the formulation F7 having the best mucoadhesion properties. The conclusion of the entire study is that only the mixture of Poloxamer 407/Poloxamer 188 in a ratio of 20:10 has the highest potential to be used as a thermosensitive gel basis for mucoadhesive formulations.

Patent application EP3127557 discloses a preparation containing ALA and poloxamer P407, that facilitates absorption of the active ingredient for topical use in photodynamic therapy.

Patent application WO 2009/105369 discloses a thermoplastic, thermoreversible pharmaceutical composition comprising a botulinum toxin and a biocompatible poloxamer that imparts thermoreversibility to the composition and additionally stabilizes botulinum toxin.

In this respect it will be appreciated that the use of a carrier consisting of a thermosensitive gel based on a mixture of poloxamers can solve the problem of ensuring controlled, gradual release of botulinum toxin, a very dangerous biological agent whose release kinetics should be controlled within very strict parameters, as well as the problem of ensuring stability of the active ingredient.

Nevertheless, it shall be noted that, according to WO 2009/105369, the product is administered by intradermal, intramuscular or subcutaneous injection and that the subsequent conversion of the active ingredient into a gel causes the formation of a kind of deposit in the tissues in which the active ingredient was injected, followed by controlled spreading of the active ingredient.

WO 2009/105369 A1 does not encompass or suggests transdermal or transmucosal administration.

WO 2009/105369 A1 does not encompass applications in which mucoadhesion have some relevance and, moreover, the pharmaceutical preparation of WO 2009/105369 A1 is by no way suitable for use in photodynamic therapy.

Patent application EP 2641590 A1 discloses a pharmaceutical composition comprising imidazoquinoline(amines) and its derivatives for treatment of bladder diseases, namely bladder tumors.

Referring to EP 2641590 A1 it will be understood that the context of use does not concern photodynamic therapy, but a pharmacological preparation having 'imidazoquinoline' or its derivatives or 'imiquimod' and derivatives as an active ingredient. The pharmaceutical preparation of EP 2641590 A1 is by no way usable in photodynamic therapy, indeed photodynamic therapy is an alternative, other from what is disclosed in EP 2641590 A1.

The object of the present invention is to provide a pharmaceutical preparation comprising a topically released active ingredient, such as 5-aminolevulinic acid and a thermosensitive carrier that affords improved adhesion to the skin and/or the mucosa, to thereby remain on the application site of application long enough to release the active ingredient, while ensuring adequate compatibility with the skin and/or the mucosa, as well as adequate sol-gel transition temperature and active ingredient release.

A further object of the present invention is to provide a pharmaceutical preparation having improved properties as discussed above and suitable for treatment of skin or mucosal pathological conditions by photodynamic therapy.

### Summary of The Invention

These objects are mainly fulfilled by a pharmaceutical preparation according to claim 1, as well as a method according to claim 9.

According to the invention, the pharmaceutical preparation comprises an active ingredient that is suitable for photodynamic therapy, and that is a photosensitizing substance, preferably 5-aminolevulinic acid and/or its salts or pharmaceutically acceptable derivatives.

Preferably, the concentration of the active ingredient, such as 5-aminolevulinic acid and/or its acid addition salts or pharmaceutically acceptable derivatives in the above preparation ranges from 1% to 10% by weight based on the total weight of the preparation.

The pharmaceutical preparation as defined above may be effectively used for treatment of diseases or lesions of the skin or the mucosa by topical administration and photodynamic therapy.

Therefore, the above objects are fulfilled by a pharmaceutical preparation as defined above for use in the treatment of a disease or of a lesion of the skin or the mucosa by photodynamic therapy.

Further characteristics and advantages of the present invention will be apparent from the following detailed description.

### Detailed Description

As used in the present description and in the appended claims, the term "gel" is intended to designate an elastic biphasic colloid material, consisting of a liquid dispersed and embedded in the solid phase. The liquid fills the structure formed by the solid, which in turn is prevented from collapsing by the surface tension of the liquid.

The term "sol-gel system" is intended to designate a colloid suspension that can solidify into a gel.

The term "carrier" is intended to designate any inert substance or mixture of inert substances, i.e. having no pharmacological activity, that can be mixed with an active ingredient for the latter to be used in the desired form, by facilitating administration and release, particularly topical administration and/or topical release, thereof.

The term "thermosensitive carrier" is intended to designate a carrier characterized by a sol-gel system in which state transition occurs above a given temperature (gelation temperature); the system is liquid below such temperature, and solid above, and the transition is reversible.

The term "poloxamer" is intended to designate a triblock copolymer consisting of a (hydrophobic) central block of poly(propylene oxide) (PPO), with two (hydrophilic) blocks of poly(ethylene oxide) (PEO) connected at its ends, that has the following general formula:

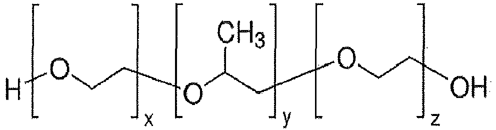

Due to the possibility of changing the length of the polymer blocks, different poloxamers may be provided, with different physico-chemical properties, designated by the letter "P" followed by three digits; the first two digits multiplied x 100 indicate the mass of the PPO component, whereas the last digit multiplied x 10 indicates the percentage of PEO. Here the pharmaceutical preparation of the invention comprises a mixture of poloxamers P407 and P188, i.e. a poloxamer in which the molecular mass of PPO is about 4000 g/mol and the PEO content is 70% (P407) and a poloxamer in which the molecular mass of PPO is about 1880 g/mol and the PEO content is 80% (P188). Considering the above general formula, the poloxamer P407 has a x and z value of 101 and a y value of 56, corresponding to approximately 70% of PEO units and 30% of PPO units, whereas the poloxamer P188 has a x and z value of 80 and a y value of 27, corresponding to approximately 80% of PEO units and 20% of PPO units. Poloxamers are also known with their trade names Synperonics^{®}, Pluronics^{®}, Kolliphor^{®}.

The terms "topical administration", "topical release" and the like are intended to designate a dosage form applied to the epithelium of the body surface of a human or animal to provide a topical therapeutic effect to application site. For example, the pharmaceutical preparation of the invention may be applied to the skin or a mucosa such as a nasal, oral, rectal, vaginal or airway mucosa.

In the present description and in the annexed claims, unless otherwise stated, percentages are expressed by weight based on the total weight of the inventive preparation.

As mentioned above, the pharmaceutical preparation of the invention contains a thermosensitive carrier comprising 19 to 24 wt% Poloxamer 407 and 4 to 8 wt% Poloxamer 188.

Preferably, the Poloxamer 407 content ranges from 21% to 23% and the Poloxamer 188 content ranges from 5% to 7%.

Thus, advantageously, the preparation of the invention has a sol-gel transition temperature that is lower than body temperature (i.e. less than about 37°C), thereby allowing application thereof in the liquid state on a surface to be treated, such as an aching (e.g. inflamed or infected) skin or mucosa, thereby minimizing the injury or mechanical stress that would result from the application of a viscous product on said aching surface.

On the other hand, upon contact with the surface to be treated, the preparation of the invention gels quickly in situ (in-situ thermosetting) thereby adhering to such surface and enabling release, for example sustained release, of the active ingredient on the surface to be treated for executing the desired therapeutic treatment, in particular by photodynamic therapy.

In addition, advantageously, at the end of desired therapeutic treatment, any excess product can be easily removed by washing with cold water, i.e. at a temperature lower than the sol-gel transition temperature, thereby avoiding the pain caused by rubbing of the treated surface.

Also, it has been surprisingly found that with the mixture of the aforementioned poloxamers in the stated ratio ranges, the preparation of the invention exhibits improved mucoadhesion properties, also as compared with similar formulations in which the content of one or both of the aforesaid poloxamers does not fall within the respective ranges provided by the present invention.

This will also improve the effectiveness of the active ingredient release, while reducing the active ingredient content with the same desired therapeutic effect.

Concerning the active ingredient, it may generally be any drug and/or active ingredient that is suitable for biological topical application to the skin or the mucosa of a human or animal where it can provide a therapeutic benefit. In particular, the present invention uses a photosensitizing substance, e.g. 5-aminolevulinic acid (ALA) as an active ingredient, to provide the desired therapeutic effect to the application site (skin or mucosa) by photodynamic therapy.

The active ingredient content in the inventive preparation may vary according to the disease under treatment, the desired therapeutic benefit, the method of treatment and/or other factors related to specific needs.

For example, the active ingredient content may range from 1% to 30%, preferably from 1% to 10%, According to the invention, the active ingredient is an ingredient that is suitable for use in photodynamic therapy, and is a photosensitive ingredient.

Preferably, the content of the ingredient suitable for use in photodynamic therapy ranges from 1% to 10%, more preferably from 4% to 6%.

In a particularly preferred embodiment, the ingredient suitable for use in photodynamic therapy is 5-aminolevulinic acid, its acid addition salts or pharmaceutically acceptable derivatives or mixtures thereof.

The acid addition salts of the 5-aminolevulinic acid preferably comprise salts with a pharmaceutically acceptable acid having a pka of less than 5, preferably a pka of less than 4, and more preferably a pka below 3. Examples of pharmaceutically acceptable acids preferably include inorganic acids such as hydrochloric acid, hydrobromic acid, sulfuric acid, phosphoric acid and in particular nitric acid, and organic acids, preferably sulfonic acid, sulfonic acid derivatives, aryl, aralkyl or naphtyl acids. Pharmaceutically acceptable derivatives include esters of 5-aminolevulinic acid, in particular alkyl esters (preferably C1-C6) such as methyl ester and hexyl ester.

The pharmaceutical preparation of the invention may further comprise, in addition to water, pharmaceutically acceptable excipients and/or additives as long as they are compatible with the active ingredient, i.e. as long as the stability and active ingredient absorption or release properties, as well as the rheological and adhesion properties of the preparation are not negatively affected.

Suitable excipients may include, for example, inert substances such as lactose, starch, magnesium stearate, calcium phosphate, mannitol, colloidal silica, microcrystalline cellulose. Suitable additives may include diluents, emulsifiers, humectants, preservatives.

Preferably, the content of each excipient or additive ranges from 0.1% to 1%, preferably from 0.1% to 0.5%.

Preferably, the preparation of the invention comprises at least a preservative whose content ranges from 0.1% to 0.5%.

According to a preferred embodiment, the preparation of the invention comprises potassium sorbate and sodium benzoate as preservatives, the content of each ranging from 0.1% to 0.5%.

If appropriate, the preparation of the invention may also comprise buffering agents such as carbonates, phosphates, acetates etc. to adjust the pH to an optimal value for use in the therapeutic treatment of the skin or the mucosa.

The preparation of the invention is for use in the treatment of skin or mucosal diseases or lesions, preferably by photodynamic therapy.

In particular, the pharmaceutical preparation of the invention may be effectively applied to the skin or a mucosa such as a nasal, oral, rectal, vaginal or airway mucosa for treatment of various diseases, preferably by photodynamic therapy. These diseases include, without limitation, selected from the group consisting of tumors and other hyperproliferative conditions such as tumors of the oral cavity, skin disorders such as psoriasis, skin cancer, actinic keratosis, infectious diseases such as bacterial, viral and fungal infections, e.g. skin ulcers (wound therapy applications), periodontitis, peri-implantitis, infections of the gingiva and the oral cavity, inflammatory diseases such as Crohn's disease, arthritis and rheumatoid arthritis, Barret esophagus and arterial restenosis.

The pharmaceutical preparation of the invention may be effectively applied to the skin or a mucosa such as a nasal, oral, rectal, vaginal or airway mucosa, also for treatment of non-pathological conditions, e.g. lesions, preferably by photodynamic therapy. These non-pathological conditions include dermatoheliosis (photoaging), acne, rosacea and other inflammatory skin diseases.

The amount of inventive preparation to be administered, the duration of the treatment etc. depend on many factors that are well within the skills of an expert physician, such as the type and severity of the inflammatory or infectious disease of the skin or the mucosa, age, sex and health condition of the patient etc.

The preparation of the invention may be prepared by a process comprising the steps of:
- dissolving the active ingredient and optionally at least one excipient and/or at least one additive, in a solvent selected from water and an aqueous buffer that has been previously cooled to 3-6°C, preferably 4°C and maintained under stirring, thereby obtaining a solution,
- adding individually predetermined amounts of Poloxamer 407 and Poloxamer 188 or mixtures thereof, to said solution under stirring at said temperature,
- stirring said solution to complete dissolution of the components.

Preferably, the active ingredient is 5-aminolevulinic acid or an acid addition salt thereof or a derivative thereof such as an ester.

Preferably, the at least a additive is potassium sorbate and sodium benzoate.

Further characteristics of the present invention will be described in the following examples which are provided by way of illustration and without limitation with reference to the figures in which:
- Figure 1 shows the fluorescence spectrum of Protoporphyrin-9 (PpIX);
- Figure 2 shows a diagram of fluorescence measures related to the concentration of PpIX in tissues treated with inventive preparations and comparative preparations versus time.

### Example 1

### Preparation of inventive preparations and comparative preparations

Pharmaceutical preparations containing the ingredients in the following Table 1 were obtained

**Table 1**

| Name | CAS number | Supplier | Remarks |
|---|---|---|---|
| HCL | 5451-09-2 | Sigma Aldrich | ALA-HCL |
| 5-aminolevulinic acid | | A-3785 | |
| Poloxamer 407 | 9003-11-6 | Sigma Aldrich | |
| | | 16758 | |
| Poloxamer 188 | 9003-11-6 | Sigma Aldrich | Kolliphor ^{®} 188 |
| | | 15759 | |
| Potassium sorbate | 24634-61-5 | Sigma Aldrich | Preservative |
| | | 85520 | E202 |
| Sodium benzoate | 532-32-1 | Sigma Aldrich | Preservative |
| | | 71300 | E211 |

The preparations were each obtained by cooling a predetermined amount of purified water at 4°C, then by optionally dissolving potassium sorbate and sodium benzoate in respective predetermined amounts in water under stirring, thereby obtaining a solution. The poloxamer 407 and optionally the poloxamer 188 were added under stirring to this solution, maintained at a temperature of 4°C, and stirring was continued to complete dissolution of the components.

This process provided six preparations with varying percentages of the aforementioned components as listed in the following Table 2, where percentages are expressed by weight based on the total weight of the preparation, the balance being composed of water.

**Table 2**

| Name Prep. | Poloxamer 407 | Poloxamer 188 | ALA-HCL | Potassium sorbate | Sodium benzoate |
|---|---|---|---|---|---|
| TE_1* | 19.00% | - | 5% | 0.15 % | 0.40 % |
| TE_2 | 19.00% | 4.00% | 5% | 0.15 % | 0.40 % |
| TE_3 | 19.00% | 8.00% | 5% | 0.15 % | 0.40 % |
| TE_4 | 24.00% | 4.00% | 5% | 0.15 % | 0.40 % |
| TE_5 | 24.00% | 4.00% | 5% | - | - |
| TE_6* | 24.00% | 2.00% | 5% | 0.15 % | 0.40 % |

| | | | | | |
|---|---|---|---|---|---|
| * Preparations NOT covered by the invention | | | | | |

### Example 2

### Effectiveness assessment

The effectiveness of the preparations of the Example 1 was assessed with reference to three quantitative parameters:
- sol-gel transition temperature
- mucoadhesion
- release of the active ingredient (δ-aminolevulinic acid).

### Sol-gel transition temperature assessment

First, it should be noted that the sol-gel transition temperature of the preparations must be less than 37°C so that the product will be in gel form in the application area.

### Material and methods

50 ml of the preparation were poured into a container placed in a thermostatically controlled environment; the container was placed on a magnetic stirrer; a magnetic bar and an immersion thermometer for registering the temperature of the product were placed in the container with the preparation.

Below the sol-gel transition temperature the bar moves freely, and above the transition temperature the bar is immobilized.

The thermostatically controlled environment was initially set to a temperature of 10°C; when the formulation reached 10°C, the temperature of the thermostatically controlled environment was constantly raised at a rate of 0.5°C/min.

The temperature recorded by the immersion thermometer when the bar stopped was noted, and considered as the sol-gel transition temperature of the formulation under test.

Measurements were repeated ten times for each preparation and the results were statistically processed (*Mathworks Matlab ANOVA1*)*.*

Sol-gel transition temperature results of the preparations under test are listed in the following Table 3

**Table.3**

| Name Preparation | Sol-gel transition temperature | Remarks |
|---|---|---|
| TE_1 | 26.5 ± 1.2 °C | |
| TE_2 | 24.0 ± 1.1 °C | |
| TE_3 | 33.2 ± 1.0 °C | |
| TE_4 | 25.8 ± 1.2 °C | |
| TE_5 | 25.2 ± 1.2 °C | Without preservatives |
| TE_6 | 28.7 ± 1.6 °C | |

As shown in Table 3, in all the preparations under test the sol-gel transition temperature is compatible with their intended use.

### Mucoadhesion assessment

The preparations in gel form were assessed for their ability to adhere to a mucosa.

### Material and methods

Disks of porcine gastric mucin were prepared (CAS number 84082-64-4 Sigma Aldrich M2378) with the following process:
1) compressing anhydrous mucin into disks having a diameter of 20 mm and a thickness of 0.5 mm using a hydraulic press Nannetti Mignon SSN/EA) with a compression force of 18 tonnes, and
2) hydrating the mucin disc before measurement with a 8% w/w solution of mucin in purified water.

Using a calibrated load cell the mucin disk was placed in contact with the surface of the gel maintained at 37°C, inside a cylindrical container.

The disk was adhered to the gel with a force of 0. 1 N for 4 minutes.

Then, the disc was listed with a speed of 0,1 mm/s and the load cell measures the value of the adhesion force as a function of distance.

The force-distance integral, up to the point of detachment of the disc, represents the work (Joule) to detach the disc from the gel and is therefore representative of the mucoadhesion of the preparation.

Measurements were repeated ten times for each preparation and the results were statistically processed.

Mucoadhesion results of the preparations under test are listed in the following Table 4

**Table.4**

| Preparation name | Detachment work |
|---|---|
| TE_1 | 270 ± 30 mJ |
| TE_2 | 650 ± 50 mJ |
| TE_3 | 820 ± 40 mJ |
| TE_4 | 840 ± 50 mJ |
| TE_5 | 820 ± 50 mJ |
| TE_6 | 520 ± 40 mJ |

As shown in Table 4, the preparations TE_2, TE_3, TE_4 and TE_5 of the invention exhibit a greater mucoadhesion as compared with the comparative preparations having one poloxamer (TE_1) only, or a mixture of the poloxamer 407 and the poloxamer 188 with contents other than those provided by the invention (TE_6).

In particular, the preparations TE_3, TE_4 and TE_5 of the invention exhibit the highest mucoadhesion values and are particularly suitable candidates for release of the active ingredient on the mucosa.

### Active ingredient release

The δ-aminolevulinic acid active ingredient is known to have no photodynamic activity by itself but is a precursor of PpIX synthesis that takes place in target tissues or in microbial populations.

Therefore, the assessment of PpIX content, that is directly related to the desired biological effects, is only of interest; for this purpose, non-invasive "in-vivo" techniques, based on the optical properties of this molecule (fluorescence), are available.

Fluorescence is a secondary light emission of light occurring when a molecule or an atom has been stimulated by absorption of electromagnetic energy and has reached a higher energy state.

Emission takes place when the molecule is restored to the base energy level; this process is typically very rapid (a few tens of nanoseconds for PpIX), whereby fluorescence ceases as soon as the excitation light is removed.

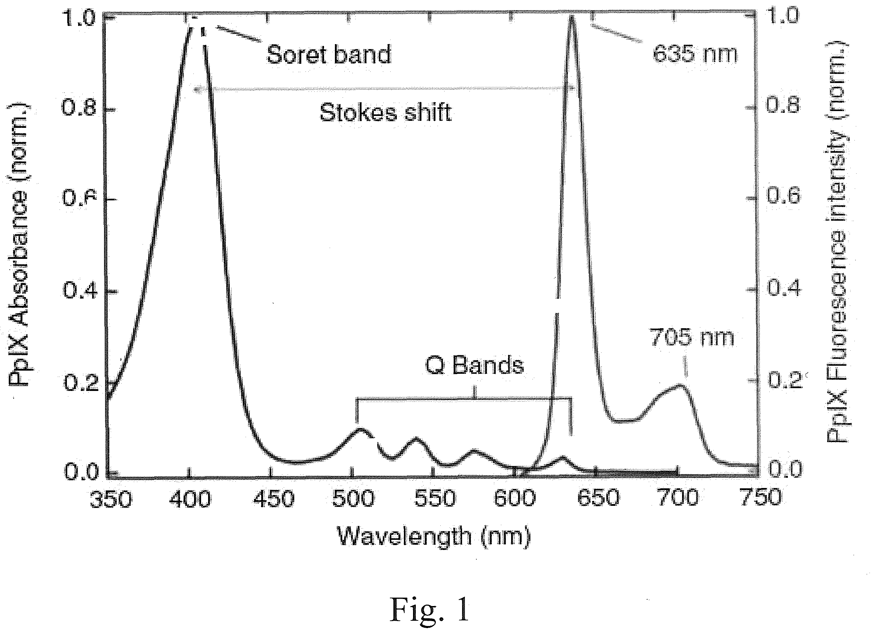

Emission has always a higher wavelength (and therefore a lower energy) than excitation; in the case of PpIX, the peak response is found around 635 nm, as shown in Figure 1.

The fluorescence of the PpIX contained in the tissues was measured with the following process:
1) the area of the skin (or the mucosa) is illuminated by a monochromatic source at 405 nm;
2) a spectroradiometer with a fiber optic probe is placed orthogonal to the area to be measured, and detects the spectrum emitted from the skin; and
3) the irradiance accumulated in the of 600-650 nm is calculated.

Fluorescence intensity is directly related to PpIX content in the tissues and therefore constitutes an effective method for 'in-vivo' assessment of the effectiveness of carriers in facilitating transport and absorption of ALA (see for example Kanick SC, Davis SC, Zhao Y, Hasan T, Maytin EV, Pogue BW, Chapman MS (2014) "Dual-channel red/blue fluorescence dosimetry with broadband reflectance spectroscopic correction measures protoporphyrin IX production during photodynamic therapy of actinic keratosis." J Biomed Opt. 2014;19(7):75002*).*

### Material and methods

### Lesion simulation

A mechanical skin stimulation condition was induced by micro-needling by means of the technique of micro-needling to create favorable conditions for PpIX synthesis.

A skin area of 10 x 1.5 cm of the of the volar left forearm region of healthy volunteers was treated with 40 longitudinal passes of a micro-needling instrument (*Dermaroller*^{®} *MC-905*).

6 rectangular 1x1 cm areas were found, on which the formulations of Example 1 were applied in an amount of 100 mg per cm². Each area was separated from the next by an interval of 1 cm in which no product was applied.

### Fluorescence detection

1) The process is carried out in a dark environment to eliminate the contributions of spurious light sources;
2) The skin area is illuminated by a monochromatic source at 405 nm *(SP-405N* - *AlphaStrumenti*);
3) A standard reflectance surface (*Ocean Optics* - *WS-1 Reflectance Standard*) and a fiber-optic spectroradiometer (*Ocean Optics* - *USB2000-UV-VIS*) are used to measure irradiance at 405 nm impinging on the surface to obtain uniform lighting conditions;
4) Fluorescence response in the 600-650 nm range (accumulated irradiance: area under the spectral curve) was measured with the fiber-optic spectroradiometer oriented orthogonal to the skin surface.

Starting from the time of application of the product, fluorescence measurement was repeated every 15 minutes up to 180 minutes and the results were statistically processed (*Mathworks Matlab ANOVA1*).

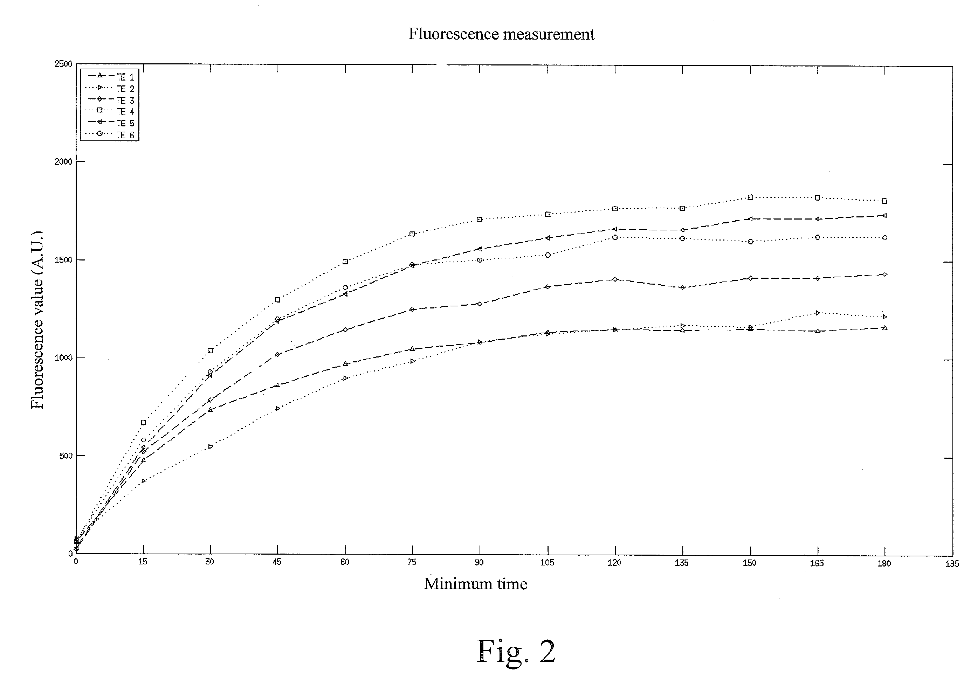

The fluorescence detection campaign involved 30 people (aged 23-78) and the results are graphically shown in Figure 2 where the curves are representative of the accumulation of PpIX in tissues versus time.

It will be appreciated that the preparations TE_4 and TE_5 have better properties than the others, as they achieve higher PpIX concentrations in a shorter time, thereby advantageously affording a reduction of the contact time as well as improved effectiveness of the therapy with the same contact time.

The formulations TE_4 and TE_5 represent an optimized trade-off with respect to the desired product characteristics.

The preparations TE_2 and TE_3, which achieve lower PpIX concentrations in the treated tissues, are still acceptable to obtain a significant therapeutic effect by photodynamic therapy.

Conversely, the comparative preparations TE_1 and TE_6, which still achieve acceptable PpIX concentrations, have poor mucoadhesion properties, as noted above, which make them inadequate for treatment of skin or mucosal infections by photodynamic therapy.

## Claims

1. A pharmaceutical preparation comprising a topically released active ingredient suitable for photodynamic therapy, that is a photosensitizing substance, and a heat-sensitive carrier comprising, by weight based on the total weight of the preparation, 19 to 24 wt% of a first block copolymer of poly(ethylene oxide)-poly(propylene oxide)- poly(ethylene-oxide) (PEO-PPO-PEO), with a molecular mass of the PPO block of about 4000 g/mol and about 70% PEO content (Poloxamer P407) and 4 to 8 wt% of a second PEO-PPO-PEO block copolymer, with a molecular mass of the PPO block of about 1800 g/mol and about 80% PEO content (Poloxamer P188), the balance being composed of water and any pharmaceutically acceptable excipients and/or additives, for use in the treatment of a skin or the mucosal disease or lesion.

2. A pharmaceutical preparation for use as claimed in claim 1, wherein said active ingredient suitable for photodynamic therapy is selected from 5-aminolevulinic acid (ALA), its acid addition salts or pharmaceutically acceptable derivatives and mixtures thereof.

3. A pharmaceutical preparation for use as claimed in claim 1 or 2, wherein the content of said first block copolymer (Poloxamer 407) ranges from 21% to 23% and the content of said second block copolymer (Poloxamer 188) ranges from 5% to 7%.

4. A pharmaceutical preparation for use as claimed in claim 1 or 2 or 3, wherein the content of the active ingredient ranges from 1% to 30%, preferably from 1% to 10%, more preferably from 4% to 6%.

5. A pharmaceutical preparation for use as claimed in any of claims 1 to 4, wherein said excipients and/or additives include at least a preservative agent, preferably potassium sorbate and sodium benzoate, whose content ranges from 0.1% to 0.5%.

6. A pharmaceutical preparation for use as claimed in claims 1-5, for use in the treatment of a skin or the mucosal disease or lesion by photodynamic therapy.

7. A pharmaceutical preparation for use as claimed in claim 6, for use on the skin or a mucous membrane such as a nasal, oral, rectal, vaginal or airway mucous membrane for treatment of any of the diseases selected from the group consisting of tumors and other hyperproliferative conditions such as tumors of the oral cavity, skin disorders such as psoriasis, skin cancer, actinic keratosis, infectious diseases such as bacterial, viral and fungal infections, e.g. skin ulcers (wound therapy applications), periodontitis, peri-implantitis, infections of the gingiva and the oral cavity, inflammatory diseases such as Crohn's disease, arthritis and rheumatoid arthritis, Barret esophagus and arterial restenosis.

8. A pharmaceutical preparation for use as claimed in claim 6, for use on the skin or a mucous membrane such as a nasal, oral, rectal, vaginal or airway mucous membrane for treatment of any of the lesions selected from the group of photoaging, acne, rosacea and other inflammatory skin diseases.

9. A method of obtaining a pharmaceutical preparation comprising a topically released active ingredient suitable for photodynamic therapy, that is a photosensitizing substance, and a heat-sensitive carrier comprising, by weight based on the total weight of the preparation, 19 to 24 wt% of a first block copolymer of poly(ethylene oxide)-poly(propylene oxide)- poly(ethylene-oxide) (PEO-PPO-PEO), with a molecular mass of the PPO block of about 4000 g/mol and about 70 % PEO content (Poloxamer P407) and 4 to 8 wt % of a second PEO-PPO-PEO block copolymer, with a molecular mass of the PPO block of about 1800 g/mol and about 80 % PEO content (Poloxamer 188), the balance being composed of water and any pharmaceutically acceptable excipients and/or additives, the method comprising the steps of:
- dissolving an active ingredient suitable for photodynamic therapy, that is a photosensitizing substance, and optionally at least an excipient and/or at least an additive, in a solvent selected from water and an aqueous buffer that has been previously cooled to 3-6°C, preferably 4°C and maintained under stirring, thereby obtaining a solution,
- adding individually predetermined amounts of Poloxamer 407 and Poloxamer 188 or mixtures thereof, to said solution under stirring at said temperature,
- stirring said solution to complete dissolution of the components.

10. A method as claimed in claim 9 wherein said active ingredient suitable for photodynamic therapy is selected from 5-aminolevulinic acid (ALA), its acid addition salts or pharmaceutically acceptable derivatives and mixtures thereof.

## Patentansprüche

1. Pharmazeutische Zubereitung, umfassend einen topisch freigesetzten Wirkstoff, der für die photodynamische Therapie geeignet ist, d. h. eine photosensibilisierende Substanz und einen wärmeempfindlichen Träger, umfassend, bezogen auf das Gesamtgewicht des Präparats, 19 bis 24 Gew.-% eines ersten Blockcopolymers aus Poly(ethylenoxid)-poly(propylenoxid)-poly(ethylenoxid) (PEO-PPO-PEO) mit einer Molekularmasse des PPO-Blocks von etwa 4000 g/mol und einem PEO-Gehalt von etwa 70 % (Poloxamer P407) und 4 bis 8 Gew.-% eines zweiten PEO-PPO-PEO-Blockcopolymers mit einer Molekularmasse des PPO-Blocks von etwa 1800 g/mol und einem PEO-Gehalt von etwa 80 % (Poloxamer P188), wobei der Rest aus Wasser und beliebigen pharmazeutisch annehmbaren Hilfsstoffen und/oder Zusatzstoffen besteht, zur Verwendung bei der Behandlung einer Haut- oder Schleimhauterkrankung oder -läsion.

2. Pharmazeutische Zubereitung zur Verwendung nach Anspruch 1, wobei der für die photodynamische Therapie geeignete Wirkstoff ausgewählt ist aus 5-Aminolävulinsäure (ALA), ihren Säureadditionssalzen oder pharmazeutisch annehmbaren Derivaten und Mischungen davon.

3. Pharmazeutische Zubereitung zur Verwendung nach Anspruch 1 oder 2, wobei der Gehalt des ersten Blockcopolymers (Poloxamer 407) im Bereich von 21 % bis 23 % und der Gehalt des zweiten Blockcopolymers (Poloxamer 188) im Bereich von 5 % bis 7 % liegt.

4. Pharmazeutische Zubereitung zur Verwendung nach Anspruch 1 oder 2 oder 3, wobei der Gehalt des Wirkstoffs im Bereich von 1 % bis 30 %, vorzugsweise von 1 % bis 10 %, noch bevorzugter von 4 % bis 6 % liegt.

5. Pharmazeutische Zubereitung zur Verwendung nach einem der Ansprüche 1 bis 4, wobei die Hilfsstoffe und/oder Zusatzstoffe mindestens ein Konservierungsmittel, vorzugsweise Kaliumsorbat und Natriumbenzoat, enthalten, dessen Gehalt im Bereich von 0,1 % bis 0,5 % liegt.

6. Pharmazeutische Zubereitung zur Verwendung gemäß den Ansprüchen 1-5 zur Verwendung bei der Behandlung einer Haut- oder Schleimhauterkrankung oder -läsion durch photodynamische Therapie.

7. Pharmazeutische Zubereitung zur Verwendung nach Anspruch 6 zur Verwendung auf der Haut oder einer Schleimhaut, wie z. B. einer Nasen-, Mund-, Rektal-, Vaginal- oder Atemwegsschleimhaut, zur Behandlung einer der Krankheiten, ausgewählt aus der Gruppe bestehend aus Tumoren und anderen hyperproliferativen Zuständen, wie z. B. Tumoren der Mundhöhle, Hauterkrankungen, wie z. B. Psoriasis, Hautkrebs, aktinische Keratose, Infektionskrankheiten, wie z. B. bakterielle, virale und Pilzinfektionen, z. B. Hautgeschwüre (Wundtherapieanwendungen), Parodontitis, Periimplantitis, Infektionen der Gingiva und der Mundhöhle, entzündliche Krankheiten, wie z. B. Morbus Crohn, Arthritis und rheumatische Arthritis, Barret-Ösophagus und arterielle Restenose.

8. Pharmazeutische Zubereitung zur Verwendung nach Anspruch 6 zur Verwendung auf der Haut oder einer Schleimhaut, wie einer Nasen-, Mund-, Rektal-, Vaginal- oder Atemwegsschleimhaut, zur Behandlung von Läsionen, ausgewählt aus der Gruppe der Lichtalterung, Akne, Rosazea und anderen entzündlichen Hauterkrankungen.

9. Verfahren zur Herstellung einer pharmazeutischen Zubereitung, umfassend einen topisch freigesetzten Wirkstoff, der für die photodynamische Therapie geeignet ist, d. h. eine photosensibilisierende Substanz, und einen wärmeempfindlichen Träger, umfassend, bezogen auf das Gesamtgewicht der Zubereitung, 19 bis 24 Gew.-% eines ersten Blockcopolymers aus Poly(ethylenoxid)-Poly(propylenoxid)-Poly(ethylenoxid) (PEO-PPO-PEO) mit einer Molekularmasse des PPO-Blocks von etwa 4000 g/mol und einem PEO-Gehalt von etwa 70 % (Poloxamer P407) und 4 bis 8 Gew.-% eines zweiten PEO-PPO-PEO-Blockcopolymers mit einer Molekularmasse des PPO-Blocks von etwa 1800 g/mol und einem PEO-Gehalt von etwa 80 % (Poloxamer 188), wobei der Rest aus Wasser und beliebigen pharmazeutisch annehmbaren Hilfsstoffen und/oder Zusatzstoffen besteht, wobei das Verfahren die folgenden Schritte umfasst:
- Lösen eines für die photodynamische Therapie geeigneten Wirkstoffs, d. h. einer photosensibilisierenden Substanz und gegebenenfalls mindestens eines Hilfsstoffs und/oder mindestens eines Zusatzstoffs, in einem Lösungsmittel, das aus Wasser und einem wässrigen Puffer ausgewählt ist, der zuvor auf 3 - 6 °C, vorzugsweise 4 °C, abgekühlt und unter Rühren gehalten wurde, wodurch eine Lösung erhalten wird,
- Zugeben von individuell vorbestimmten Mengen von Poloxamer 407 und Poloxamer 188 oder Mischungen davon zu der Lösung unter Rühren bei der genannten Temperatur,
- Rühren der Lösung bis zum vollständigen Auflösen der Bestandteile.

10. Verfahren nach Anspruch 9, wobei der für die photodynamische Therapie geeignete Wirkstoff ausgewählt ist aus 5-Aminolävulinsäure (ALA), ihren Säureadditionssalzen oder pharmazeutisch annehmbaren Derivaten und Mischungen davon.

## Revendications

1. Préparation pharmaceutique comprenant un principe actif à libération par voie topique approprié pour la thérapie photodynamique, c'est-à-dire une substance photosensibilisante et un support thermosensible comprenant, en poids par rapport au poids total de la préparation, 19 à 24 % en poids d'un premier copolymère séquencé de poly(oxyde d'éthylène)-poly(oxyde de propylène)-poly(oxyde d'éthylène) (PEO-PPO-PEO), avec une masse moléculaire du bloc PPO d'environ 4 000 g/mol et une teneur en PEO d'environ 70 % (Poloxamère P407) et 4 à 8 % en poids d'un second copolymère séquencé PEO-PPO-PEO, avec une masse moléculaire du bloc PPO d'environ 1 800 g/mol et une teneur en PEO d'environ 80 % (Poloxamère P188), le reste étant composé d'eau et de tout excipient et/ou additif pharmaceutiquement acceptable, pour une utilisation dans le traitement d'une maladie ou d'une lésion de la peau ou des muqueuses.

2. Préparation pharmaceutique destinée à être utilisée selon la revendication 1, dans laquelle ledit ingrédient actif approprié pour la thérapie photodynamique est choisi parmi l'acide 5-aminolévulinique (ALA), ses sels d'addition d'acide ou ses dérivés pharmaceutiquement acceptables et leurs mélanges.

3. Préparation pharmaceutique destinée à être utilisée selon la revendication 1 ou 2, dans laquelle la teneur dudit premier copolymère séquencé (Poloxamère 407) est comprise entre 21 % et 23 % et la teneur dudit second copolymère séquencé (Poloxamère 188) est comprise entre 5 % et 7 %.

4. Préparation pharmaceutique destinée à être utilisée selon la revendication 1 ou 2 ou 3, dans laquelle la teneur en principe actif est comprise entre 1 % et 30 %, de préférence entre 1 % et 10 %, plus préférablement entre 4 % et 6 %.

5. Préparation pharmaceutique destinée à être utilisée selon l'une quelconque des revendications 1 à 4, dans laquelle lesdits excipients et/ou additifs comportent au moins un agent conservateur, de préférence le sorbate de potassium et le benzoate de sodium, dont la teneur est comprise entre 0,1 % et 0,5 %.

6. Préparation pharmaceutique destinée à être utilisée selon les revendications 1 à 5 pour une utilisation dans le traitement d'une maladie ou d'une lésion de la peau ou des muqueuses par thérapie photodynamique.

7. Préparation pharmaceutique destinée à être utilisée selon la revendication 6, pour une utilisation sur la peau ou une membrane muqueuse telle qu'une membrane muqueuse nasale, orale, rectale, vaginale ou des voies respiratoires pour le traitement de l'une quelconque des maladies choisies dans le groupe constitué des tumeurs et autres affections hyperprolifératives telles que les tumeurs de la cavité orale, les troubles de la peau tels que le psoriasis, le cancer de la peau, la kératose actinique, les maladies infectieuses telles que les infections bactériennes, virales et fongiques, par exemple, les ulcères cutanés (applications de thérapie des plaies), la parodontite, la péri-implantite, les infections de la gencive et de la cavité buccale, les maladies inflammatoires telles que la maladie de Crohn, l'arthrite et la polyarthrite rhumatoïde, l'œsophage de Barret et la resténose artérielle.

8. Préparation pharmaceutique destinée à être utilisée selon la revendication 6, pour une utilisation sur la peau ou une membrane muqueuse telle qu'une membrane muqueuse nasale, orale, rectale, vaginale ou des voies respiratoires pour le traitement de l'une quelconque des lésions choisies dans le groupe du photovieillissement, de l'acné, de la rosacée et d'autres maladies inflammatoires de la peau.

9. Procédé d'obtention d'une préparation pharmaceutique comprenant un principe actif à libération par voie topique approprié pour la thérapie photodynamique, c'est-à-dire une substance photosensibilisante et un support thermosensible comprenant, en poids par rapport au poids total de la préparation, 19 à 24 % en poids d'un premier copolymère séquencé de poly(oxyde d'éthylène)-poly(oxyde de propylène)- poly(oxyde d'éthylène) (PEO-PPO-PEO), avec une masse moléculaire du bloc PPO d'environ 4 000 g/mol et une teneur en PEO d'environ 70 % (Poloxamère P407) et 4 à 8 % en poids d'un second copolymère séquencé PEO-PPO-PEO, avec une masse moléculaire du bloc PPO d'environ 1 800 g/mol et une teneur en PEO d'environ 80 % (Poloxamère 188), le reste étant composé d'eau et de tout excipient et/ou additif pharmaceutiquement acceptable, le procédé comprenant les étapes suivantes :
- la dissolution d'un principe actif approprié pour la thérapie photodynamique, c'est-à-dire une substance photosensibilisante et éventuellement au moins un excipient et/ou au moins un additif, dans un solvant choisi parmi l'eau et un tampon aqueux qui a été préalablement refroidi entre 3 et 6 °C, de préférence 4 °C et maintenu sous agitation, obtenant ainsi une solution,
- l'ajout de quantités individuellement prédéterminées de Poloxamère 407 et de Poloxamère 188 ou de leurs mélanges, à ladite solution sous agitation à ladite température,
- l'agitation de ladite solution pour achever la dissolution des composants.

10. Procédé selon la revendication 9, dans lequel ledit principe actif approprié pour la thérapie photodynamique est choisi parmi l'acide 5-aminolévulinique (ALA), ses sels d'addition d'acide ou ses dérivés pharmaceutiquement acceptables et leurs mélanges.
